# EUROPEAN PATENT APPLICATION

(11) **EP 4 649 962 A1**
(43) Date of publication of application: **19.11.2025**
(21) Application number: 23915194.7
(22) Date of filing: 11.07.2023
(51) Int. Cl.: A61K 47/34, A61K 9/127, A61K 9/14

(54) **METHOD FOR EVADING PREEXISTING ANTI-PEG ANTIBODY IN HUMAN BODY**

(30) Priority: 10.01.2023 CN 202310031365
(71) Applicant: Fudan University, Shanghai 200433 (CN)
(72) Inventor: ZHAN, Changyou, Shanghai 200031 (CN); DING, Tianhao, Shanghai 200032 (CN)
(74) Representative: Epping - Hermann - Fischer
(86) International application number: PCT/CN2023/106753
(87) International publication number: WO 2024/148785

(57) **Abstract**

The present invention discloses a method for circumventing pre-existing anti-PEG antibodies in the human body and the use of a terminal hydroxyl-modified PEGylated nanocarrier in the preparation of a drug that circumvents pre-existing anti-PEG antibodies in the human body. The present invention also discloses a terminal hydroxyl-modified PEGylated nanocarrier and a nanoformulation comprising the same. The terminal hydroxyl-modified PEGylated nanocarrier and nanoformulation according to the present invention have a low binding to pre-existing anti-PEG antibodies in the human body, so that they can circumvent being rapidly cleared in the human blood and better exert the therapeutic effect. In addition, the terminal hydroxyl-modified PEGylated nanocarrier and nanoformulation can reduce complement activation by circumventing a binding to pre-existing anti-PEG antibodies in human blood, thereby alleviating side effects such as clinical injection reactions.

## Description

### FIELD OF THE INVENTION

The present invention belongs to the field of biomedical technology and relates to a method for circumventing pre-existing anti-PEG antibodies in the human body. Specifically, the present invention relates to a terminal hydroxyl-modified PEGylated nanocarrier and a nanoformulation comprising the same, and a use of the terminal hydroxyl-modified PEGylated nanocarrier in circumventing pre-existing anti-PEG antibodies in the human body.

### BACKGROUND OF THE INVENTION

Pre-existing anti-polyethylene glycol (PEG) antibodies in the human body refer to anti-PEG antibodies detected in normal people or clinical patients who have not been administrated PEG-containing drugs or drug carriers. A study by Kozma et al. showed that the current production mechanism of pre-existing anti-PEG antibodies was unknown, which may be closely related to the widespread application of PEG as an additive in food or cosmetics, and over time, the positive rate of anti-PEG antibodies in the population increased from 0.2% in 1984 to 40% in 2016 (Kozma, G.T., et al., Anti-PEG antibodies: Properties, formation, testing and role in adverse immune reactions to PEGylated nano-biopharmaceuticals. Advanced Drug Delivery Reviews, 2020. 154-155: p. 163-175; Richter, A.W. and E. Akerblom, Polyethylene glycol reactive antibodies in man: titer distribution in allergic patients treated with monomethoxy polyethylene glycol modified allergens or placebo, and in healthy blood donors. Int Arch Allergy Appl Immunol, 1984. 74(1): p. 36-9; Chen, B.-M., et al., Measurement of Pre-Existing IgG and IgM Antibodies against Polyethylene Glycol in Healthy Individuals. Analytical Chemistry, 2016. 88(21): p. 10661-10666).

PEG is widely used in the field of nanomedicine because it can increase *in vivo* and *in vitro* stabilities and *in vivo* long-cycle characteristics of nanocarriers or nanoformulations modified by it. In the formulations currently on the market, the end groups of PEG are basically methoxy groups. For example, the PEGylated lipid structure in the PEGylated liposome encapsulating doxorubicin (Doxil^{®}) is methoxy polyethylene glycol-distearoylphosphatidylethanolamine; the PEGylated lipid structure used in the marketed siRNA drug lipid nanoparticle Onpattro and the mRNA COVID-19 vaccine produced by Moderna is methoxy polyethylene glycol-dimyristoylglycerol; and the PEGylated lipid structure used in the mRNA COVID-19 vaccine produced by BioNTech is methoxy polyethylene glycol-tetracosyl acetamide. One of the reasons for using a methoxy group at one end of PEG is because methoxy PEG (mPEG) is structurally simple and inert, so that only one active end of PEG can be derivatized, resulting in minimal cross-linking in the synthesis reaction and the highest homogeneity in the final product.

However, recent studies have found that PEG improves the performance of nanocarriers or nanoformulations *in vivo,* but there are also some problems that cannot be ignored, for example, anti-PEG antibodies *in vivo* rapidly recognize and bind to PEGylated nanocarriers or nanoformulations, activating the complement system, resulting in the opsonization of complement C3 fragments on particles, thereby accelerating the uptake of PEGylated nanocarriers or nanoformulations by the body's mononuclear macrophage system (MPS) and making them quickly clear from the blood. Anti-PEG antibodies are the main cause of accelerated clearance from the blood, and clinical experience with PEGylated therapies has shown that anti-PEG antibodies not only can increase clearance and lose efficacy, but also cause side effects such as severe injection reactions.

In addition to the above-mentioned pre-existing anti-PEG antibodies *in vivo* (the source mechanism is unknown, but the anti-PEG antibodies are already present in the body prior to administration), anti-PEG antibodies may also be produced after repeated injections of PEGylated nanocarriers. For example, if PEGylated nanocarriers or nanoformulations are injected into animal models such as mice, rats, guinea pigs, rabbits, beagles, rhesus monkeys, etc., anti-PEG antibodies can be detected in the body after 1 week, and are mainly anti-PEG IgM antibodies, which show a significant downward trend after 2-3 weeks *in vivo.* It has been reported that the anti-PEG IgM antibodies are mainly produced after stimulating the proliferation and differentiation of B cells in the marginal zone of the spleen in a T cell-independent (TI) manner, and are generally produced by a lower dose of PEGylated nanocarriers, while a high dose of PEGylated nanocarriers or those carrying chemotherapy drugs (such as doxorubicin) produce lower or no anti-PEG antibodies (Koide, H., et al., T cell-independent B cell response is responsible for ABC phenomenon induced by repeated injection of PEGylated liposomes. Int J Pharm, 2010. 392(1-2): p. 218-23).

The anti-PEG antibody produced by repeated dosing and its effect on PEGylated nanocarriers or nanoformulations *in vivo* are highly controversial. On one hand, the dose of most PEGylated nanocarriers for producing anti-PEG antibodies by repeated injections is often lower than the dose for clinical application, and the clinical relevance is expected to be poor. On the other hand, PEGylated nanocarriers currently in clinical use do not produce antibodies when used for cytotoxic drug delivery. Finally, there are significant species differences in the anti-PEG antibodies produced by repeated injections, resulting in different effects on the *in vivo* performance of PEGylated nanocarriers or nanoformulations, which affects their effective evaluation of clinical transformation (Suzuki, T., et al., Influence of dose and animal species on accelerated blood clearance of PEGylated liposomal doxorubicin. Int J Pharm, 2014. 476(1-2): p. 205-12). In order to solve the impact of repeated injections of anti-PEG antibodies in animals on PEGylated nanocarriers or nanoformulations, some researchers have tried to replace PEG with PVP, PDMA, HPMA and other materials, and many researchers have invested a lot of time in the structural modification of PEG. The Chinese patent application CN113350512A records that PEG is immunogenic, and it is proposed to directly solve the immunogenicity of PEG. Specifically, compared with PEG2k (linear PEG), the first injection of PEG2,n (branched PEG)-modified nanocarriers into rats resulted in a significant reduction in the production of anti-PEG antibodies, suggesting that the use of PEG2,n-modified nanocarriers can reduce the production of anti-PEG antibodies. In addition, the first injection of PEG2,n-modified emulsion and repeated injections after seven days led to a different level of binding of the formulation to the antibody. With the increase of PEG2,n molecular weight, the binding of the formulation to the antibody slowed down, and the binding of PEG2,40k to the antibody was the slowest. These results suggest that high molecular weight PEG2,n modified nanocarriers are more conducive to prolonging the cycle time of nanocarriers, and PEG2,n-lipid derivative modified nanocarriers can solve the problems of accelerated clearance of PEGylated nanocarriers in blood and a significant increase in the aggregation in liver and spleen.

U.S. patent application No. US20210046188A1 reports the use of branched polymers containing PEG side chains to reduce or eliminate the antigenicity of molecules and eliminate their reactivity to patient-derived PEG antibodies. The patent application discloses that the end group of the PEG side chain is a methoxy group, and states that the key to eliminating the pre-existing anti-PEG antibody is the length of the PEG side chain and the end group of the PEG side chain.

Shimizu et al. reported that the first dose of PEGylated liposomes (PL) elicited an anti-PEG IgM antibody response that triggered a rapid systemic clearance of the second dose of PL through a phenomenon called "accelerated blood clearance (ABC)" (Shimizu, T., et al., A hydroxyl PEG version of PEGylated liposomes and its impact on anti-PEG IgM induction and on the accelerated clearance of PEGylated liposomes. European Journal of Pharmaceutics and Biopharmaceutics, 2018. 127: p. 142-149). This reference confirms in mice that PL-OH essentially activates the complement system by the alternative pathway in the absence of anti-PEG IgM. In the presence of anti-PEG IgM, PL-OH activates the complement system not only through the classical pathway mediated by anti-PEG IgM, but also through the alternative pathway. Therefore, due to this strong complement-activating property, PL-OH may be rapidly cleared from the blood circulation even in the absence (first dose) or presence (second dose) of anti-PEG IgM.

### SUMMARY OF THE INVENTION

### Technical Problems to be Solved by the Present Invention

The inventor has found in a long-term research on PEGylated nanocarriers that:
1. The biological activity of anti-PEG antibodies produced by repeated administration of PEGylated nanocarriers in animal models is significantly different from that of pre-existing anti-PEG antibodies in human bodies (the source mechanism is unknown, but the anti-PEG antibodies are already present in the body prior to administration), and the binding activities of pre-existing anti-PEG antibodies in human bodies to PEGs with different end groups are different. The inventors investigated the commonly used PEG containing methoxy end groups (MeO-PEG), PEG containing carboxyl end groups (HOOC-PEG), PEG containing amino end groups (H₂N-PEG) and PEG containing hydroxyl end groups (HO-PEG), and found that the pre-existing anti-PEG antibody in the human body had the highest binding activity to MeO-PEG and the lowest binding activity to HO-PEG. Because the methoxy PEG is structurally simple and the methoxy group is inert, only one active end in PEG can be derivatized, so that the cross-linking degree of the synthesis reaction is the smallest, and the homogeneity of the final product is the highest. Therefore, the PEGylated nanoformulations currently on the market are basically MeO-PEG derivatives. The inventor found that the binding mechanism of anti-PEG antibody produced by injection of PEGylated nanocarriers in animals is different from that of pre-existing anti-PEG antibodies in humans, and the main antigen binding site of anti-PEG antibodies produced by PEGylated nanocarriers in rats may be ethylene glycol repeat units in the PEG chain, which is not selective for PEG end groups, so the anti-PEG antibodies produced in rats have similar high binding activities to the structures with different PEG end groups. However, the pre-existing anti-PEG antibodies in human body mainly recognize PEG end groups, so the pre-existing anti-PEG antibodies in human serum have different binding activities to the structures with different PEG end groups, and the binding activity with HO-PEG is the lowest. Most drugs require animal testing before clinical trials, and the inventors speculate that due to the lack of recognition of the different binding mechanisms of antibodies, the fact that anti-PEG antibodies produced in animals have the same binding activity with the structures with different PEG end groups has prevented researchers from applying HO-PEGylated nanocarriers to human research and further development of human drugs, while focusing more on inert MeO-PEGylated nanocarriers and nanoformulations.
2. Sherman et al. found that proteins modified by PEG containing hydroxyl end groups produced lower immunogenicity and better *in vivo* performance in rabbits compared with proteins modified by PEG containing methoxy end groups (Sherman, M.R., et al., Role of the Methoxy Group in Immune Responses to mPEG-Protein Conjugates. Bioconjugate Chemistry, 2012. 23(3): p. 485-499). However, Shimizu et al. (*supra*) found that liposomes modified by PEG containing hydroxyl end groups enhanced complement activation, resulting in stronger accelerated clearance after secondary injection compared to liposomes modified by PEG containing methoxy end groups. Based on the contradictory results of PEG modification containing hydroxyl end groups in animals, the inventor investigated the complement activation capabilities of various nanocarriers modified by PEG containing hydroxyl end groups in bloods of different animal species and human (as shown in Example 3), and found that liposomes modified by PEG containing hydroxyl end groups had certain signs of enhanced complement activation in mice and rats, but no enhanced complement activation was seen in human blood. When the nanocarriers were lipid nanoparticles, the complement activation capability of PEG modification containing hydroxyl end groups in mouse and rat bloods is comparable to that of PEG modification containing methoxy end groups, which means that the effect of PEG modification containing hydroxyl end groups on the complement activation capability of nanocarriers or nanoformulations is closely related to the species and the type of nanocarriers. None of the nanocarriers detected in human blood showed significant enhancement of complement activation, indicating that the animal experimental results reported in the literature hinder the application of terminal hydroxyl-modified PEGylated nanocarriers or nanoformulations in human research.

Based on the above findings, the purpose of the present invention is to apply a HO-PEGylated nanocarrier with low binding activity with anti-PEG antibody in human body to a nanoformulation for human use, and to provide a PEGylated nanoformulation that can circumvent binding to pre-existing anti-PEG antibodies in human and circumvent rapid clearance *in vivo* and side effects such as injection reaction.

### Technical Solutions for Solving Technical Problems

The invention provides a use of a terminal hydroxyl-modified PEGylated nanocarrier in the preparation of a drug that circumvents pre-existing anti-PEG antibodies in the human body, wherein the PEGylated nanocarrier is selected from one or more of liposomes, polymer nanoparticles, micelles and lipid nanoparticles.

In an embodiment, the molecular weight of the PEG is 500-10,000 Da. In an embodiment, the molecular weight of the PEG is 1,000-5,000 Da. In an embodiment, the molecular weight of the PEG is 2,000 Da.

In an embodiment, the ratio of PEG modification in the liposomes and lipid nanoparticles is 0.5mol%-10mol%, preferably 1mol%-5mol%.

In an embodiment, the ratio of PEG modification in the polymer nanoparticles and micelles is 0.5mol%-100mol%, preferably 50mol%-100mol%.

In an embodiment, the drug includes one or more of small molecule drugs, protein drugs and nucleic acid drugs.

In an embodiment, the nucleic acid drugs are selected from one or more of small interfering ribonucleic acids (siRNA), deoxyribonucleic acids (DNA) and messenger ribonucleic acids (mRNA).

In an embodiment, the terminal hydroxyl-modified PEG has a hydroxyl group at one end, and a polymer such as an amphiphilic polymer or lipid at the other end.

In an embodiment, the PEGylated nanocarrier includes one or more of hydroxyl polyethylene glycol polylactic acid-glycolic acid copolymer (HO-PEG-PLGA), hydroxyl polyethylene glycol polylactic acid copolymer (HO-PEG-PLA), hydroxyl polyethylene glycol-distearoylphosphatidylethanolamine (HO-PEG-DSPE) and hydroxyl polyethylene glycol-dimyristoylglycerol (HO-PEG-DMG).

The invention also provides a nanoformulation for human use. In an embodiment, the nanoformulation comprises an active ingredient and a terminal hydroxyl-modified PEGylated nanocarrier. In an embodiment, the nanocarrier is selected from one or more of liposomes, polymer nanoparticles, micelles and lipid nanoparticles (LNP). In an embodiment, the active ingredient is selected from one or more of small molecule drugs, protein drugs and nucleic acid drugs.

In an embodiment, the molecular weight of PEG in the nanoformulation for human use of the present invention is 500Da-10,000Da.

In an embodiment, in the nanoformulation for human use of the present invention, the ratio of PEG modification in the liposomes and lipid nanoparticles is 0.5mol%-10mol%, preferably 1mol%-5mol%. In an embodiment, the ratio of PEG modification in the micelles and polymer nanoparticles is 0.5mol%-100mol%, preferably 50mol%-100mol%.

In an embodiment, in the nanoformulation for human use of the present invention, the nucleic acid drugs are selected from one or more of siRNA, DNA and mRNA.

In an embodiment, in the nanoformulation for human use of the present invention, the terminal hydroxyl-modified PEG has a hydroxyl group at one end, and a polymer such as an amphiphilic polymer or lipid at the other end.

In an embodiment, in the nanoformulation for human use of the present invention, the PEGylated nanocarrier includes one or more of hydroxyl polyethylene glycol polylactic acid-glycolic acid copolymer (HO-PEG-PLGA), hydroxyl polyethylene glycol polylactic acid copolymer (HO-PEG-PLA), hydroxyl polyethylene glycol-distearoylphosphatidylethanolamine (HO-PEG-DSPE) and hydroxyl polyethylene glycol-dimyristoylglycerol (HO-PEG-DMG).

The invention also provides a method for treating a disease by circumventing pre-existing anti-PEG antibodies in the human body, wherein the method includes administering a nanoformulation to a human subject in need thereof, wherein the nanoformulation comprises an active ingredient and a terminal hydroxyl-modified PEGylated nanocarrier. In an embodiment, the nanocarrier is selected from one or more of liposomes, polymer nanoparticles, micelles and lipid nanoparticles (LNPs). In an embodiment, the active ingredient is selected from one or more of small molecule drugs, protein drugs and nucleic acid drugs.

In an embodiment, the terminal hydroxyl-modified PEGylated nanocarrier is able to reduce complement activation.

In an embodiment, the molecular weight of the PEG is 500-10,000 Da. In an embodiment, the molecular weight of the PEG is 1,000-5,000 Da. In an embodiment, the molecular weight of the PEG is 2,000 Da.

In an embodiment, the ratio of PEG modification in the liposomes and lipid nanoparticles is 0.5mol%-10mol%, preferably 1mol%-5mol%.

In an embodiment, the ratio of PEG modification in the polymer nanoparticles and micelles is 0.5mol%-100mol%, preferably 50mol%-100mol%.

In an embodiment, the nucleic acid drugs are selected from one or more of siRNA, DNA and mRNA.

In an embodiment, the terminal hydroxyl-modified PEG has a hydroxyl group at one end, and a polymer such as an amphiphilic polymer or lipid at the other end.

In an embodiment, the PEGylated nanocarrier includes one or more of hydroxyl polyethylene glycol polylactic acid-glycolic acid copolymer (HO-PEG-PLGA), hydroxyl polyethylene glycol polylactic acid copolymer (HO-PEG-PLA), hydroxyl polyethylene glycol-distearoylphosphatidylethanolamine (HO-PEG-DSPE) and hydroxyl polyethylene glycol-dimyristoylglycerol (HO-PEG-DMG).

### Beneficial Technical Effects

The terminal hydroxyl-modified PEGylated nanocarrier and nanoformulation according to the present invention have a low binding to pre-existing anti-PEG antibodies in the human body, so that they can circumvent being rapidly cleared in the human blood and better exert the therapeutic effect. In addition, the terminal hydroxyl-modified PEGylated nanoformulation according to the present invention can reduce complement activation by circumventing a binding to pre-existing anti-PEG antibodies in human blood, thereby circumventing side effects such as clinical injection reactions.

### BRIEF DESCRIPTION OF THE DRAWINGS

Figure 1 shows a comparison between the bindings of anti-PEG antibodies to MeO-PEG and HO-PEG as test antigens in human blood samples.
Figure 2 shows the amount of anti-PEG-IgM produced by weekly repeated injections of HO-PEG or MeO-PEG modified nanocarriers into mice, in which a, b, c, and d are the results of serum experiments in mice unstimulated, after one week, three weeks, and six weeks, respectively.
Figure 3 shows the complement activation results of HO-PEG or MeO-PEG modified nanocarriers in different animal and human serums.
Figure 4 shows the binding activity of anti-PEG-IgM produced in rat serum and pre-existing anti-PEG-IgM in human serum to DSPE with different PEG end groups, in which a is the experimental result of rat serum, and b is the experimental result of human serum.
Figure 5 shows the binding activity of pre-existing anti-PEG-IgM in human serum to HO-PEG with different hydrophobic groups or PEG chain lengths, in which a, b, c, and d represent the experimental results of hydrophobic groups and PEG chain lengths of PEG2000-DSPE, PEG2000-DMG, PEG10000-PLGA, and PEG500-DMG, respectively.
Figure 6 shows the results of the binding activity of pre-existing anti-PEG-IgM in human serum to blank liposomes with different proportions of modified PEG, in which a, b, and c are the experimental results of 1%, 3%, and 5% of modified PEG, respectively.
Figure 7 shows the binding activity of pre-existing anti-PEG-IgM in human serum to HO-PEG liposomes encapsulating different drugs, in which a and b are the experimental results of encapsulated albumin and doxorubicin, respectively.
Figure 8 shows the binding activity of pre-existing anti-PEG-IgM in human serum to HO-PEG micelles and MeO-PEG micelles.
Figure 9 shows the binding activity of pre-existing anti-PEG-IgM in human serum to HO-PEG polymer nanoparticles and MeO-PEG polymer nanoparticles.
Figure 10 shows the binding activity of pre-existing anti-PEG-IgM in human serum to LNPs with different proportions of modified PEG, in which a, b, c, d, e, and f are the experimental results of 5% modified DSPE end group, 3% modified DSPE end group, 1.5% modified DSPE end group, 5% modified DMG end group, 3% modified DMG end group, and 1.5% modified DMG end group, respectively.
Figure 11 shows the binding activity of pre-existing anti-PEG-IgM in human serum to LNPs encapsulating siRNA.
Figure 12 shows the binding activity of pre-existing anti-PEG-IgM in human serum to LNPs encapsulating mRNA.
Figure 13 shows the binding activity of pre-existing anti-PEG-IgM in human serum to LNPs encapsulating DNA.
Figure 14 shows the complement activation results of PEGylated nanoformulations.
Figure 15 shows the complement activation results of PEGylated liposomes and lipid nanoparticles.

### DETAILED DESCRIPTION OF THE INVENTION

The purpose of the present invention is to apply a HO-PEGylated nanocarrier with a low binding activity with a pre-existing anti-PEG antibody in the human body to a human drug, and to provide a PEGylated nanoformulation that can circumvent binding to the pre-existing anti-PEG antibody in the human body and avoid being rapidly cleared in the human body.

The inventors experimentally verified a variety of end-modified PEGylated nanocarriers that are commonly used at present, and found that HO-PEGylated liposomes, polymer nanoparticles, micelles, and lipid nanoparticles (LNPs) exhibited a low binding activity with pre-existing anti-PEG antibodies in human body.

In the present invention, the molecular weight of PEG is preferably 500-10,000Da, more preferably 1,000-5,000Da, and from the perspective of easy to obtain, most preferably 2,000Da.

In the liposomes and lipid nanoparticles, the ratio of PEG modification is 0.5mol%-10mol%. In the polymer nanoparticles and micelles, the ratio of PEG modification is 0.5mol%-100mol%.

In the present invention, the terminal hydroxyl-modified PEG has a hydroxyl group at one end, and any structure that facilitates embedding in the nanocarrier at the other end. The structure that facilitates embedding in the nanocarrier does not participate in antibody binding, but only for the purpose of realizing the modification of the nanocarrier by PEG, and therefore is not a critical factor for the practice of the present invention. Such structure can be a polymer, such as an amphiphilic polymer, or a lipid. Such structure includes, but are not limited to, one or more of polylactic acid-glycolic acid copolymer, polylactic acid, distearoylphosphatidylethanolamine, dimyristoylglycerol, phosphatidylglycerol, phosphatidylserine, phosphatidylinositol, phosphatidylethanolamine, phosphatidylcholine, lecithin, cardiolipin, and sphingomyelin. In an embodiment, the fatty acid chains of phosphatidylglycerol, phosphatidylserine, phosphatidylinositol, phosphatidylethanolamine and phosphatidylcholine are between 4 and 28 carbons in length and have an unsaturation of 0 to 3.

In the present invention, the drug is not limited and may be any one or more of small molecule drugs, protein drugs and nucleic acid drugs. Among them, the nucleic acid drug is not particularly limited, and can be any one or more of siRNA, DNA and mRNA.

In the present invention, the lipid is not limited, and various commonly used lipids on the market can be used, such as one or more of dimyristoylglycerol, distearoylphosphatidylethanolamine, polylactic acid-glycolic acid, soybean lecithin, egg yolk lecithin, phosphatidylglycerol, EPG, phosphatidic acid, cardiolipin, sphingomyelin, phosphatidylserine, phosphatidylinositol, phosphatidylethanolamine, hydrogenated soy lecithin, hydrogenated egg yolk lecithin, distearoylphosphatidylcholine, dipalmitoylphosphatidylcholine, dioleoylphosphatidylcholine, dimyristoylphosphatidylcholine, dilauroylphosphatidylcholine, didecanoylphosphatidylcholine, dicapryloylphosphatidylcholine, dihexanoylphosphatidylcholine, distearoylphosphatidylglycerol and its salts, dipalmitoylphosphatidylglycerol and its salts, L-α-dimyristoylphosphatidylglycerol and its salts, dilauroylphosphatidylglycerol, didecanoylphosphatidylglycerol, dicapryloylphosphatidylglycerol, dihexanoylphosphatidylglycerol, dipalmitoylphosphatidylethanolamine, dioleoylphosphatidylethanolamine, dimyristoylphosphatidylethanolamine, dilauroylphosphatidylethanolamine, bis-distearoylphosphatidylglycerol and its salts, bis-dipalmitoylphosphatidylglycerol and its salts, bis-dimyristoylphosphatidylglycerol and its salts, bis-dilauroylphosphatidylglycerol, distearoylphosphatidylinositol, dipalmitoylphosphatidylinositol, dioleoylphosphatidylinositol, dimyristoylphosphatidylinositol, dilauroylphosphatidylinositol, palmitoyloleoylphosphatidylcholine, palmitoyl-linoleoylphosphatidylcholine, stearoyllinoleoylphosphatidylcholine, stearoyl-oleoylphosphatidylcholine and stearoylarachidonylphosphatidylcholine.

### EXAMPLE

The present invention is described in detail below in conjunction with examples. The examples enumerate the detailed embodiments of the present invention for fully disclosing and demonstrating that the invention is implementable. However, the examples do not represent a limitation of the present invention, and the scope of protection of the present invention is limited by the claims. Based on the specific examples of the present invention, a person skilled in the art may obtain all aspects and scope of the invention without inventive labor.

In the examples of the present invention, unless otherwise specified, all raw material components are commercially available products well known to those skilled in the art; PEG derivatives used in the examples, including but not limited to MeO-PEG₂₀₀₀-DSPE, HO-PEG₂₀₀₀-DSPE, NH₂-PEG₂₀₀₀-DSPE, COOH-PEG₂₀₀₀-DSPE, MeO-PEG₂₀₀₀-DMG, HO-PEG₂₀₀₀-DMG, are purchased from A.V.T. (Shanghai) Pharmaceutical Technology Co., Ltd.; MeO-PEG₁₀₀₀₀-PLGA (50:50) and HO-PEG₁₀₀₀₀-PLGA (50:50) are purchased from Xi'an Ruixi Biotechnology Co., Ltd. In the examples of the present invention, if not specified, the technical means used are conventional means well known to those skilled in the art. The process of the reaction of the present invention can be monitored by conventional monitoring methods in the art (such as TLC, HPLC, LCMS or NMR), and the reaction reaches the end point generally when the reaction substrate disappears.

The experimental methods in the examples that do not specify the specific conditions are usually in accordance with the conventional conditions in the art or in accordance with the conditions recommended by the manufacturer. In the present invention, unless otherwise specified, "part" means a part by weight, and "%" denotes a molar percentage. In the present invention, unless otherwise specified, "above", "below", and "within" means including this number.

### Abbreviations:

| | |
|---|---|
| MeO-PEG₂₀₀₀-DSPE | distearoylphosphatidylethanolamine-polyethylene glycol 2000-methoxy end-capped |
| HO-PEG₂₀₀₀-DSPE | distearoylphosphatidylethanolamine-polyethylene glycol 2000-hydroxy end-capped |
| NH₂-PEG₂₀₀₀-DSPE | distearoylphosphatidylethanolamine-polyethylene glycol 2000-amino end-capped |
| COOH-PEG₂₀₀₀-DSPE | distearoylphosphatidylethanolamine-polyethylene glycol 2000-carboxy end-capped |
| MeO-PEG₂₀₀₀-DMG | dimyristoylglycerol-polyethylene glycol 2000-methoxy end-capped |
| HO-PEG₂₀₀₀-DMG | dimyrisoylglycerol-polyethylene glycol 2000-hydroxy end-capped |
| MeO-PEG₁₀₀₀₀-PLGA (50:50) | polylactic acid-glycolic acid-polyethylene glycol 10000-methoxy end-capped |
| HO-PEG₁₀₀₀₀-PLGA (50:50) | polylactic acid-glycolic acid-polyethylene glycol 10000-hydroxy end-capped |
| sLip/DOX | liposomes encapsulating doxorubicin |
| sLip/OVA | liposomes encapsulating ovalbumin |
| LNP/mRNA | lipid nanoparticles encapsulating mRNA |
| PM/PTX | micelles encapsulating paclitaxel |
| NP/PTX | PLGA nanoparticles encapsulating paclitaxel |
| DSPC | distearoylphosphatidylcholine |

Unless otherwise specified, HO-PEG-DSPE means HO-PEG₂₀₀₀-DSPE, and MeO-PEG-DSPE means MeO-PEG₂₀₀₀-DSPE.

### Explanation of terms

Small molecule drugs: mainly refer to chemically synthesized drugs, usually organic compounds with a molecular weight of less than 1,000.

Ratio of PEG modification: the molar ratio of PEGylated material to the total material of the formulation or carrier.

### Example 1

The present example is used to illustrate that the pre-existing anti-PEG antibody in the human body has a high binding activity to MeO-PEG.

Sandwich ELISA was used to detect the proportion of people who were positive for the pre-existing anti-PEG antibodies. The particular steps were as follows.

Step (1): 2 µg of MeO-PEG₂₀₀₀-DSPE was added to each well of a medium-binding ELISA plate, and kept overnight at room temperature. After three washes in PBST, the plate was blocked with 5% BSA for 1 h at 37°C. After three washes in PBST, serial dilutions of human serum in 1wt% BSA-PBS (4-fold dilution in the first well) were added, and incubated at 37°C for 1.5 h. After three washes in PBST, horseradish peroxidase-labeled anti-rat or human IgM antibody was added, and after 1 h it reacted with TMB chromogenic solution for 8 min. The reaction was terminated with 0.18 M H₂SO₄, and its absorbance value was measured at 450 nm wavelength. It was considered to be a positive sample of MeO-PEG if the OD value of the first well was greater than 1.0.

Step (2): 2 µg of HO-PEG₂₀₀₀-DSPE was added to each well of a medium-binding ELISA plate, and kept overnight at room temperature. After three washes in PBST, the plate was blocked with 5wt% BSA for 1 h at 37°C. After three washes in PBST, serial dilutions of human serum in 1% BSA-PBS (serum from the MeO-PEG-IgM-positive population screened in step (1) above) were added, and incubated at 37°C for 1.5 h. After three washes in PBST, horseradish peroxidase-labeled anti-rat or human IgM antibody was added, and after 1 h it reacted with TMB chromogenic solution for 8 min. The reaction was terminated with 0.18 M H₂SO₄, and its absorbance value was measured at 450 nm wavelength. It was considered to be a positive sample of HO-PEG if the OD value of the first well was greater than 1.0.

854 clinical blood samples were screened for the pre-existing antibody, and it was found that about 25% of the blood samples had a strong binding activity with MeO-PEG (ELISA test, OD>1.0). HO-PEG was replaced as the test antigen, and these samples that were positive for MeO-PEG were tested, and the results showed that only about 10% were positive for HO-PEG, which means that the pre-existing antibodies in human blood samples were selective for MeO-PEG. The pre-existing anti-PEG antibodies in human body have a high binding activity with MeO-PEG and a low binding activity with HO-PEG.

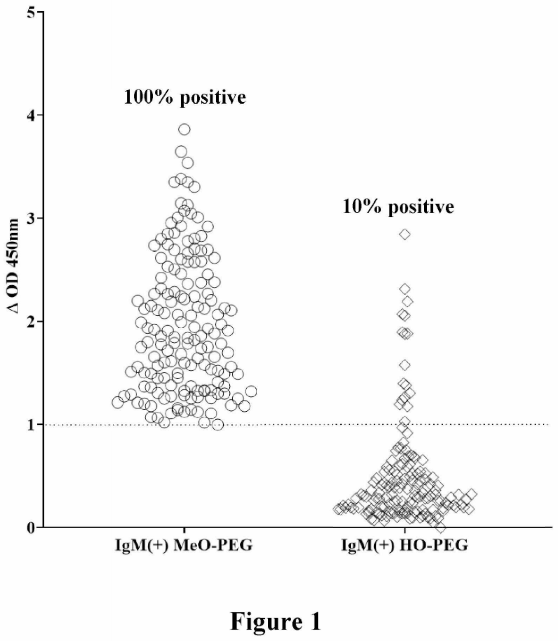

The results are shown in Figure 1.

### Example 2

The present example is used to illustrate that the immunogenicity of the HO-PEG-DSPE-modified nanoformulation is lower than that of MeO-PEG-DSPE. The particular steps were as follows.

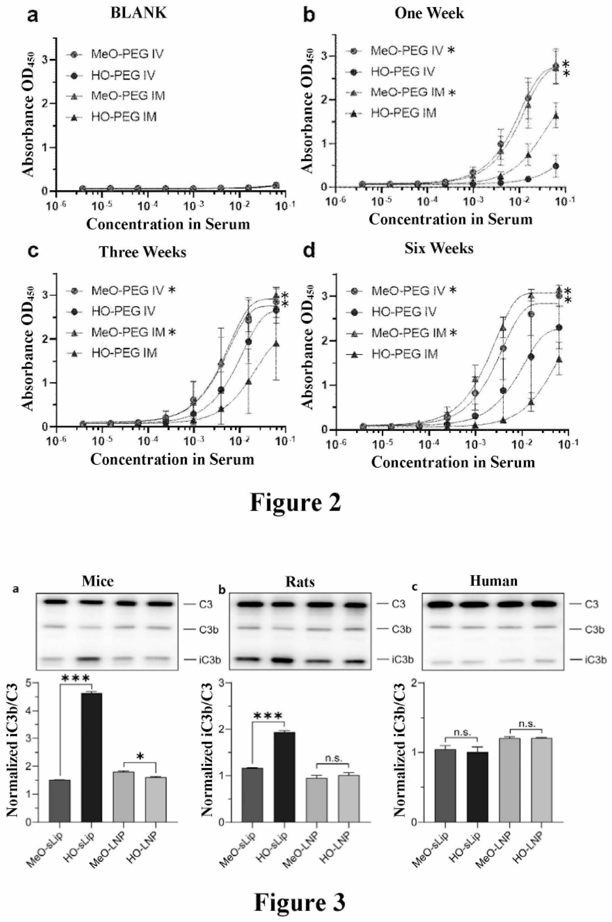

MeO-PEG-DSPE- or HO-PEG-DSPE-modified LNPs were repeatedly injected into mice (10 mg LNP/kg, once a week) intravenously (IV) or intramuscularly (IM). The serum of mice was taken before the first injection, 1 week, 3 weeks and 6 weeks after the first injection, respectively and the amount of anti-PEG-IgM produced by stimulating mice was detected by sandwich ELISA. 2 µg of MeO-PEG₂₀₀₀-DSPE or HO-PEG₂₀₀₀-DSPE (corresponding to the injected formulations) was added to each well of a medium-binding ELISA plate, and kept overnight at room temperature. After three washes in PBST, the plate was blocked with 5% BSA for 1 h at 37°C. After three washes in PBST, serial dilutions of mouse serum in PBS were added, and incubated at 37°C for 1 h. After three washes in PBST, horseradish peroxidase-labeled anti-mouse IgM antibody was added, and after 1 h it reacted with TMB chromogenic solution for 10 min. The reaction was terminated with 0.18 M H₂SO₄, and its absorbance value (OD value) was measured at 450 nm wavelength. The experimental results are shown in Figure 2.

By comparing the immunogenicity of PEG-modified LNPs containing hydroxyl or methoxy end groups in mice, it was found that the anti-PEG-IgM produced by PEG-modified LNPs with hydroxyl end groups in mice was significantly lower than that of PEG-modified LNPs with methoxy end groups, regardless of tail vein injection or intramuscular injection, and the results were similar between single injection (1 injection, Fig. 2, b) and multiple injections (2 injections, Fig. 2, c; 5 injections, Figure 2, d). This means that the PEG-modified nanoformulation with hydroxyl end groups has the advantage of low immunogenicity.

### Example 3

The present example is used to illustrate that the effect of PEG modification containing hydroxyl end groups on the complement activation capability of nanocarriers or nanoformulations is closely related to species and nanocarrier types. The particular steps were as follows.

Liposomes or LNPs modified with PEG containing hydroxyl or methoxy end groups were mixed with mouse, rat, or human serum, and incubated at 37°C for 1.5 h. The reaction was terminated with 10 mM EDTA-PBS, and the serum was diluted 500-fold. The loading buffer was added and incubated at 95°C for 10 min to denature the proteins. The proteins were separated by molecular weight by 4-20% sodium dodecyl sulfate-polyacrylamide gel electrophoresis (SDS-PAGE), and then the cleavage degree of C3 protein in serum was detected by western blotting, and the ratio of lysate to C3 protein was counted.

In order to compare the effect of PEG modification containing hydroxyl end groups on the complement activation capability of liposomes or LNPs, the cleavage of C3 protein was detected by western blotting after incubation of formulations with serum. The cleavage of C3 protein is a necessary pathway for complement activation and thus can be an indication of complement activation capacity. As shown in Figure 3, PEG-modified liposomes with hydroxyl end groups showed some signs of enhanced complement activation in mice and rats, in comparison with methoxy-terminated PEG modification, but no enhanced complement activation was seen in human serum, and when the nanocarrier was a lipid nanoparticle, its complement activation capability in mouse and rat serum was comparable to that of methoxy-terminated PEG modification. None of the nanocarriers detected in human serum showed significant enhancement of complement activation, which means that the effect of hydroxyl-terminated PEG modification on the complement activation capability of nanocarriers or nanoformulations is closely related to species and nanocarrier types, and the results in animals cannot reflect the real situation in humans.

### Example 4

The present example is used to illustrate that the anti-PEG-IgM produced by stimulation of rats has the same binding activity to the structures with different PEG end groups, while the pre-existing anti-PEG-IgM in human serum has different binding activity to the structures with different PEG end groups (all are end groups-PEG-DSPE structures), in which the binding activity is the lowest when the end group is HO.

A low dose of PEGylated liposomes (5 mg HSPC/kg) was injected into rats to stimulate the production of anti-PEG-IgM, and when the PEG antibody content peaked on day 6, rat serum was taken to compare its binding pattern with that of pre-existing anti-PEG-IgM in healthy human serum.

Sandwich ELISA was used to detect the binding activity of anti-PEG-IgM produced by stimulating rats and pre-existing anti-PEG-IgM in human serum with different PEG end group structures. The particular steps were as follows.

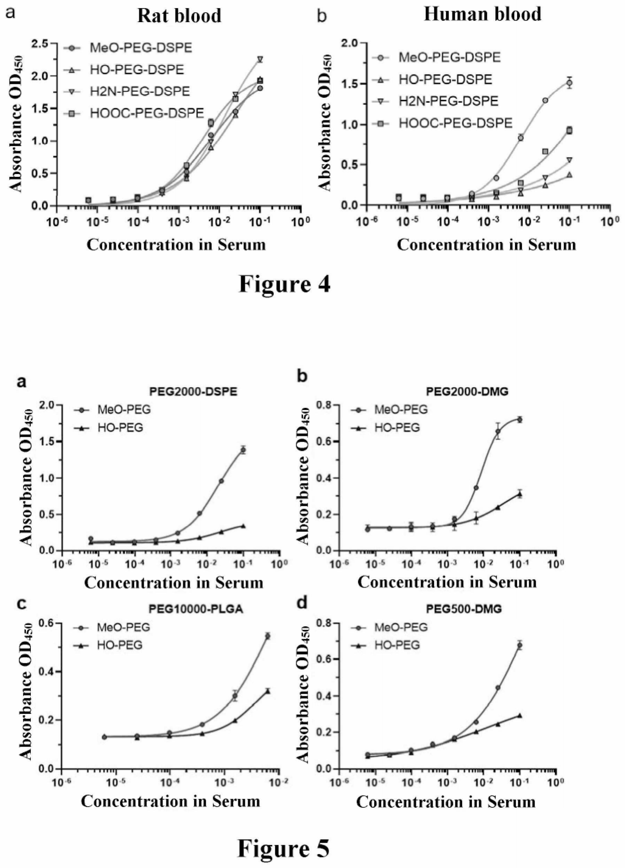

2 µg of MeO-PEG₂₀₀₀-DSPE, HO-PEG₂₀₀₀-DSPE, H₂N-PEG₂₀₀₀-DSPE, and HOOC-PEG₂₀₀₀-DSPE were added to each well of the ELISA plate, and kept overnight at room temperature. After three washes in PBST, the plate was blocked with 5% BSA for 1 h at 37°C. After three washes in PBST, serial dilutions of rat or human serum in PBS were added, and incubated at 37°C for 1 h. After three washes in PBST, horseradish peroxidase-labeled anti-rat or human IgM antibody was added, and after 1 h it reacted with TMB chromogenic solution for 10 min. The reaction was terminated with 0.18 M H₂SO₄, and its absorbance value (OD value) was measured at 450 nm wavelength. The experimental results are shown in Figure 4.

In order to compare the PEG binding patterns between the anti-PEG-IgM produced by PEG drug stimulation in animals and the pre-existing anti-PEG-IgM in humans, PEG liposomes were injected into rats through the tail vein, blood was taken 6 days later, serum was separated, and the binding activity of anti-PEG-IgM in rat serum and healthy human serum with different PEG end group structures was detected by sandwich ELISA. As shown in Figure 4a, rat serum has the same binding activity with the four PEGylated materials. After analysis, it is believed that this is due to the fact that the main antigen binding site of anti-PEG-IgM produced by PEGylated drugs in rats is the PEG chain, which is basically non-selective for PEG end groups, so it has the same binding activity with different end groups. As shown in Figure 4b, human serum has the strongest binding to MeO-modified PEG, followed by carboxyl-modified PEG and amino modified PEG, and almost no binding to HO-modified PEG. After analysis, it is believed that this is due to the fact that the pre-existing anti-PEG-IgM in the human body mainly recognizes PEG end groups. This result proves that the binding mechanism of anti-PEG-IgM produced by injection of PEGylated drugs in animals is different from that of pre-existing anti-PEG antibodies in humans, and the pre-existing anti-PEG-IgM in humans has different binding activities to different PEG end group structures, in which the binding activity with HO end group structures is the lowest.

### Example 5

The present example is used to illustrate that the pre-existing anti-PEG-IgM in human serum has a low binding activity to different HO-PEGs, independent of hydrophobic groups or PEG chain lengths (comparison of MeO-PEG and HO-PEG; grafted DSPE, DMG and PLGA; PEG molecular weights of 500, 2,000 and 10,000).

The binding activity of pre-existing anti-PEG-IgM in human serum with PEG linked to different hydrophobic groups was detected by sandwich ELISA. 2 µg of MeO-PEG₂₀₀₀-DSPE, HO-PEG₂₀₀₀-DSPE, MeO-PEG₂₀₀₀-DMG, HO-PEG₂₀₀₀-DMG, MeO-PEG₁₀₀₀₀-PLGA, HO-PEG₁₀₀₀₀-PLGA, MeO-PEG₅₀₀-DMG, and HO-PEG₅₀₀-DMG were added to each well of the ELISA plate. The method of Example 4 was used to detect the binding of different materials and anti-PEG-IgM. The experimental results are shown in Figure 5.

From the results of Example 4, it can be seen that the terminal HO-modified PEG₂₀₀₀-DSPE material can circumvent the binding of anti-PEG-IgM, so the binding activity of other commonly used PEG materials and PEG-IgM was further detected to investigate whether the low binding advantage of HO-PEG was related to the hydrophobic group or PEG molecular weight. PEG-DMG is often used to prepare lipid nanoparticles encapsulating nucleic acid drugs (such as COVID-19 mRNA-LNP vaccines on the market), and PEG-PLGA is often used to prepare nanoparticles encapsulating chemotherapy drugs. After changing the end group MeO of the two materials to HO, the binding ability of the two materials to anti-PEG-IgM was detected by ELISA. As shown in Figure 5, the binding capability of HO end groups to anti-PEG-IgM was significantly weaker than that of MeO end groups for PEG-modified DMG and PLGA materials, in addition to DSPE. When the PEG chain length was 500, 2,000 and 10,000, the capability of terminal hydroxyl modified PEG to bind IgM was weaker than that of terminal methoxy modified PEG. Therefore, the low binding activity of pre-existing anti-PEG-IgM in human serum to different HO-PEGs was independent of hydrophobic groups or the molecular weight of PEG chains, and had universality.

### Example 6

This example is used to illustrate that the binding activity of pre-existing anti-PEG-IgM in human serum to HO-PEG liposomes is independent of the PEG modification ratio.

Liposomes in three PEG modification ratios were prepared as shown in Table 1:

**Table 1**

| Membrane material | HSPC (mg) | CHO (mg) | MeO-PEG₂₀₀₀-DSPE(mg) | HO-PEG₂₀₀₀-DSPE(mg) |
|---|---|---|---|---|
| 5%MeO-sLip | 9.58 | 3.19 | 3.19 | 0 |
| 5%HO-sLip | 9.58 | 3.19 | 0 | 3.19 |
| 3%MeO-sLip | 9.58 | 3.19 | 1.914 | 0 |
| 3%HO-sLip | 9.58 | 3.19 | 0 | 1.914 |
| 1%MeO-sLip | 9.58 | 3.19 | 0.638 | 0 |
| 1%HO-sLip | 9.58 | 3.19 | 0 | 0.638 |

The membrane material was dissolved in 5 mL of chloroform. The membrane was formed by rotary evaporation at 60°C, and vacuum dried to remove the organic solvent. 1 mL of double distilled water was added to the membrane in a 60°C water bath for uniform hydration, and liposomes with different MeO-PEG or HO-PEG modification ratios were obtained by extrusion through 200 nm and 100 nm pore size filter membranes in the liposome extruder.

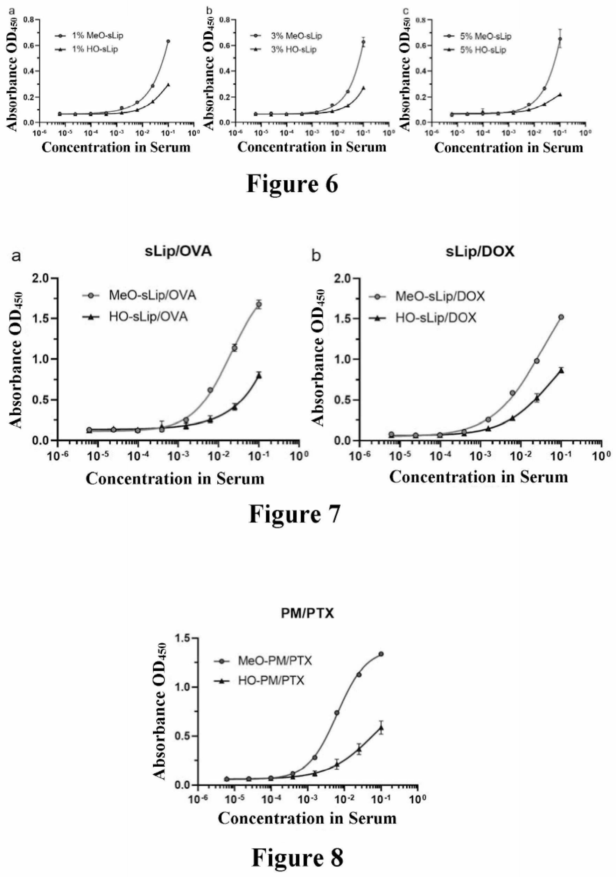

20 µg of liposomes in PBS were added to each well of the ELISA plate and the plate was coated overnight at 4°C. Then the binding activity of liposomes in different PEG modification ratios to pre-existing anti-PEG-IgM in human serum was detected by sandwich ELISA, as shown in Figure 6.

Blank liposomes in different PEG modification ratios were prepared, and their binding activity with anti-PEG-IgM was investigated. As shown in Figure 6, the binding activity of HO-PEG liposomes to pre-existing anti-PEG-IgM in human serum was significantly lower than that of MeO-PEG liposomes, regardless of the PEG modification ratio of 1%, 3%, or 5%. It can be concluded that HO-PEG liposomes have a low binding activity with PEG-IgM, independent of the PEG modification ratio.

### Example 7

This example is used to illustrate that the binding activity of pre-existing anti-PEG-IgM in human serum to HO-PEG liposomes is independent of the type of drug encapsulated.

5% PEG-modified liposomes encapsulating doxorubicin (small molecule drug, sLip/DOX) and ovalbumin (protein antigen, sLip/OVA) were prepared respectively as follows:
sLip/DOX: a uniform lipid membrane containing 5% MeO-PEG or HO-PEG was obtained according to the method of Example 6. 1 mL of 0.25 M ammonium sulfate aqueous solution was added, and the membrane was uniformly hydrated in a 60°C water bath. The liposomes hydrated by ammonium sulfate solution were obtained by extrusion through 200 nm and 100 nm pore sizes in the liposome extruder. After the liposome outer aqueous phase was replaced with normal saline by the dextran gel G-50 column, doxorubicin aqueous solution (drug-to-lipid ratio 1:10) was added. The unencapsulated doxorubicin was removed by a G50 chromatography column to obtain sLip/DOX.
sLip/OVA: a uniform lipid membrane containing 5% MeO-PEG or HO-PEG was obtained according to the method of Example 6. 1 mL of 1mg/mL OVA aqueous solution was added to hydrate the liposome. The liposomes hydrated by the OVA aqueous solution were obtained by extrusion through 200 nm and 100 nm pore sizes in the liposome extruder. The unencapsulated OVA was removed by the dextran gel G-75 column, and the outer aqueous phase was replaced with normal saline.

20 µg of liposomes in PBS was added to each well of the ELISA plate, and the plate was coated overnight at 4°C. Then the binding activity of liposomes encapsulating different drugs to pre-existing anti-PEG-IgM in human serum was detected by sandwich ELISA, as shown in Figure 7.

MeO-PEG or HO-PEG modified liposomes were prepared, which encapsulated different drugs - a small molecule chemotherapy drug doxorubicin and a protein antigen drug ovalbumin. The liposomes were coated on ELISA plates as antigens (20 µg formulation/well). Their binding to natural anti-PEG-IgM in human serum was detected by sandwich ELISA. As shown in Figure 7, the binding activity of HO-PEG liposomes encapsulating different drugs to pre-existing anti-PEG-IgM in human serum was lower than that of MeO-PEG liposomes. That is, the low binding activity of HO-PEG liposomes with pre-existing anti-PEG-IgM was not related to the encapsulated drugs.

### Example 8

This example is used to illustrate that pre-existing anti-PEG-IgM in human serum has a low binding activity to HO-PEG micelles.

Micelles (PM) consisting of MeO-PEG2000-DSPE or HO-PEG2000-DSPE encapsulating paclitaxel (PTX) were prepared respectively. The method was as follows: 10 mg of MeO-PEG2000-DSPE or HO-PEG2000-DSPE and 1 mg paclitaxel were weighed and dissolved in 5 mL of chloroform. The membrane was formed by rotary evaporation at 60°C, and the organic solvent was removed by vacuum drying. 1 mL of normal saline solution was added so that the membrane was uniformly hydrated in a 60°C water bath. MeO paclitaxel micelles (MeO-PM/PTX) and HO paclitaxel micelles (HO-PM/PTX) were obtained by filtering through a 0.22 µm filter membrane. MeO-PM/PTX and HO-PM/PTX were added to the wells of ELISA plates at 20 µg lipid/well, respectively, and their binding activity to pre-existing anti-PEG-IgM in human serum was detected as shown in Figure 8.

In order to demonstrate that the micelles containing HO-PEG material have a lower binding activity to pre-existing anti-PEG-IgM in human serum, micelles consisting of MeO-PEG2000-DSPE or HO-PEG2000-DSPE encapsulating paclitaxel were prepared and added to the wells of ELISA plates at 20 µg/well to detect their binding activity with pre-existing anti-PEG-IgM in human serum. As shown in Figure 8, the pre-existing anti-PEG-IgM in human serum had a lower binding activity to HO-PM/PTX, that is, the effect of HO-PEG material to circumvent the binding of PEG-IgM still exists in micelles.

### Example 9

This example is used to illustrate that the pre-existing anti-PEG-IgM in human serum has a low binding activity to HO-PEG polymer nanoparticles.

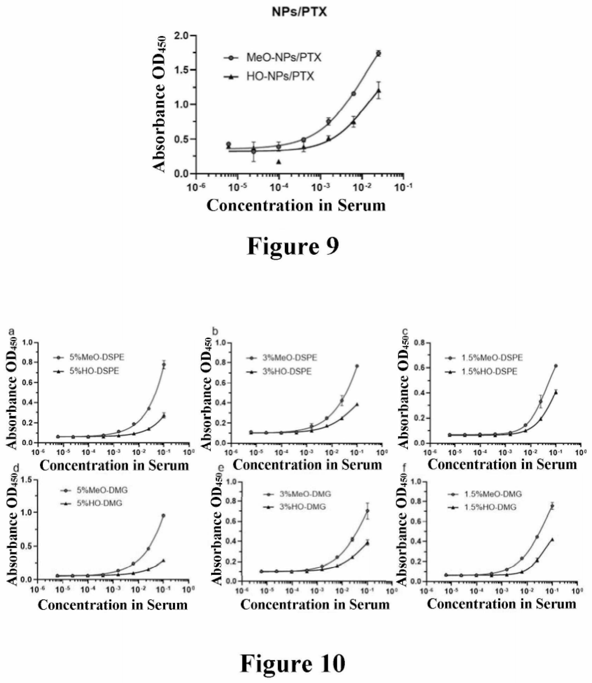

Polymer nanoparticles consisting of MeO-PEG10000-PLGA or HO-PEG10000-PLGA encapsulating paclitaxel were prepared. The method was as follows: 10 mg PLGA, 10 mg MeO-PEG10000-PLGA or HO-PEG10000-PLGA and 1 mg paclitaxel were weighed and dissolved in 1 mL of dichloromethane. 4 mL of 0.5% sodium cholate aqueous solution was added, and emulsified by ultrasound at 34% (650W) power for 8 min. The organic solvent was removed by rotary evaporation, and the large particles were removed by centrifugation at 3000 rpm. The small particles were removed by centrifugation at 12000 rpm. Homogeneous paclitaxel nanoparticles were obtained and dispersed in normal saline. MeO-NPs/PTX and HO-NPs/PTX were added to the wells of ELISA plates at 20 µg/well, respectively, and their binding activity to pre-existing anti-PEG-IgM in human serum was detected as shown in Figure 9.

In order to demonstrate that polymer nanoparticles containing HO-PEG have a low binding activity to pre-existing anti-PEG-IgM in human serum, nanoparticles consisting of MeO-PEG10000-PLGA or HO-PEG10000-PLGA encapsulating paclitaxel were prepared and added to the wells of ELISA plates at 20 µg/well to detect their binding activity with pre-existing anti-PEG-IgM in human serum. As shown in Figure 9, the pre-existing anti-PEG-IgM in human serum has a lower binding activity to HO-NPs/PTX, that is, the effect of HO-PEG material to circumvent the binding of PEG-IgM still exists in polymer nanoparticles.

### Example 10

This example is used to illustrate that the pre-existing anti-PEG-IgM in human serum has a low binding activity to HO-PEG/LNP (lipid nanoparticles), and to compare MeO-PEG and HO-PEG in different modification ratios.

Solid lipid nanoparticles (LNPs) in different PEG modification ratios were prepared as shown in Table 2:

**Table 2**

| Membrane material | SM-102 (mg) | DSPC (mg) | CHO(mg) | MeO-PEG2000-DSPE/DMG(mg) | HO-PEG2000-DSPE/DMG(mg) |
|---|---|---|---|---|---|
| 1.5%MeO-LNP | 6.1 | 0.136 | 2.56 | 0.66 | 0 |
| 1.5%HO-LNP | 6.1 | 0.136 | 2.56 | 0 | 0.66 |
| 3%MeO-LNP | 6.1 | 0.136 | 2.56 | 1.31 | 0 |
| 3%HO-LNP | 6.1 | 0.136 | 2.56 | 0 | 1.31 |
| 5%MeO-LNP | 6.1 | 0.136 | 2.56 | 2.23 | 0 |
| 5%HO-LNP | 6.1 | 0.136 | 2.56 | 0 | 2.23 |

The membrane material was dissolved in 2 mL of ethanol, mixed with 6.25 mM sodium acetate buffer solution in a microfluidic instrument (PDMS chip) at a volume and flow rate ratio of 1:3, and then the 6x volume of pre-cooled PBS was added to dilute the stable system, and then the LNPs of HO/MeO-PEG2000-DSPE and HO/MeO-PEG2000-DMG in different PEG modification ratios were obtained by ultrafiltration concentration.

200 µg of LNP in PBS was added to each well of the ELISA plate, and the plate was coated at 4°C overnight, and then the binding activity of LNPs in different PEG modification ratios to pre-existing anti-PEG-IgM in human serum was detected by sandwich ELISA, as shown in Figure 10.

Solid lipid nanoparticles with different PEG-DSPE or PEG-DMG modification ratios were prepared, and their binding activity with anti-PEG-IgM was investigated. As shown in Figure 10, although the modified PEGs carried different hydrophobic groups (DSPE/DMG), all HO-LNPs showed a lower binding to PEG-IgM, and the change in PEG modification ratio did not affect the low binding activity of pre-existing anti-PEG-IgM in human serum to HO-LNPs. That is, HO-LNP has a lower binding activity with PEG-IgM, independent of the PEG modification ratio or the hydrophobic group carried by the modified PEG.

### Example 11

This example is used to illustrate that the pre-existing anti-PEG-IgM in human serum has a low binding activity to HO-PEG/LNP encapsulating siRNA.

Since Example 10 proves that the lower binding activity of HO-LNP with PEG-IgM is independent of the PEG modification ratio or the hydrophobic group carried by the modified PEG, 3% PEG-DMG-modified LNPs encapsulating siRNA were selected to detect the binding activity with anti-PEG-IgM. The method was as follows: 6.1 mg SM-102, 0.136 mg DSPC, 2.56 mg CHO, and 1.31 mg MeO-PEG2000-DMG or HO-PEG2000-DMG were weighed and dissolved in 2 mL ethanol and mixed with 6.25 mM sodium acetate buffer solution (containing ApoB siRNA 10 µg/mL) in a microfluidic instrument (PDMS chip) at a volume and flow rate ratio of 1:3. Then the 6x volume of pre-cooled PBS was added to dilute the stable system, and then the LNPs encapsulating siRNA (HO/MeO-LNP/siRNA) were obtained by ultrafiltration concentration.

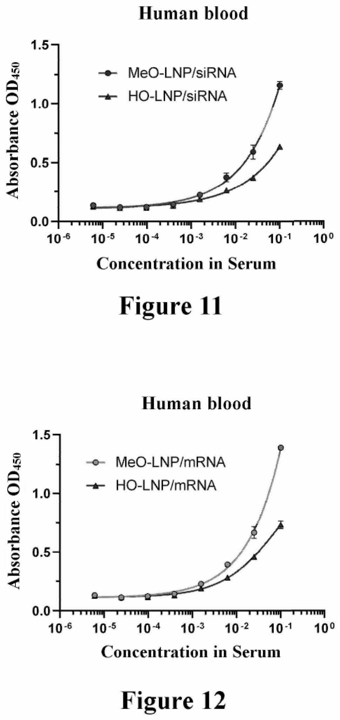

200 µg of LNP in PBS was added to each well of the ELISA plate, and the plate was coated at 4°C overnight. Then the binding activity of LNPs encapsulating siRNA to pre-existing anti-PEG-IgM in human serum was detected by sandwich ELISA, as shown in Figure 11.

Solid lipid nanoparticles encapsulating siRNA were prepared and their binding activity to anti-PEG-IgM was investigated. As shown in Figure 11, the HO-LNP/siRNA showed a lower PEG-IgM binding. That is, after encapsulating the siRNA, HO-LNP still retains a lower binding activity to PEG-IgM.

### Example 12

This example is used to illustrate that the pre-existing anti-PEG-IgM in human serum has a low binding activity to HO-PEG/LNP encapsulating mRNA.

3% PEG-DMG-modified LNPs were selected to encapsulate mRNA for detecting the binding activity with anti-PEG-IgM. The method was as follows: 6.1 mg SM-102, 0.136 mg DSPC, 2.56 mg CHO, and 1.31 mg MeO-PEG-DMG or HO-PEG-DMG were weighed and dissolved in 2 mL ethanol and mixed with 6.25 mM sodium acetate buffer solution (containing Luciferase mRNA 10 µg/mL) in a microfluidic instrument (PDMS chip) at a volume and flow rate ratio of 1:3. Then, the 6x volume of pre-cooled PBS was added to dilute the stable system, and then the LNPs encapsulating mRNA (HO/MeO-LNP/mRNA) were obtained by ultrafiltration concentration.

200 µg of LNP in PBS was added to each well of the ELISA plate, and the plate was coated at 4°C overnight. Then the binding activity of LNPs encapsulating firefly luciferase mRNA to pre-existing anti-PEG-IgM in human serum was detected by sandwich ELISA, as shown in Figure 12.

Solid lipid nanoparticles encapsulating firefly luciferase mRNA were prepared, and their binding activity with anti-PEG-IgM was investigated. As shown in Figure 12, HO-LNP/mRNA showed a lower binding to PEG-IgM. That is, after encapsulating mRNA, HO-LNP still retains a lower binding activity with PEG-IgM.

### Example 13

This example is used to illustrate that the pre-existing anti-PEG-IgM in human serum has a low binding activity to HO-PEG/LNP encapsulating DNA.

3% PEG-DMG-modified LNPs were selected to encapsulate DNA for detecting the binding activity with anti-PEG-IgM. The method was as follows: 6.1 mg SM-102, 0.136 mg DSPC, 2.56 mg CHO, and 1.31 mg MeO-PEG-DMG or HO-PEG-DMG were weighed and dissolved in 2 mL ethanol and mixed with 6.25 mM sodium acetate buffer solution (containing PTEN DNA 10 µg/mL) in a microfluidic instrument (PDMS chip) at a volume and flow rate ratio of 1:3. Then, the 6x volume of pre-cooled PBS was added to dilute the stable system, and then the LNPs encapsulating DNA (HO/MeO-LNP/DNA) were obtained by ultrafiltration concentration.

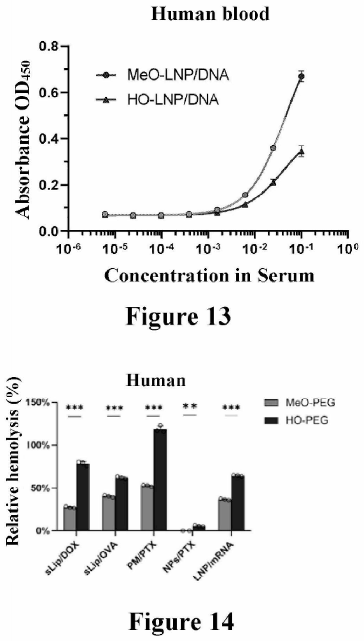

200 µg of LNP in PBS was added to each well of the ELISA plate, and the plate was coated at 4°C overnight. Then the binding activity of LNPs encapsulating the tumor suppressor gene PTEN DNA to pre-existing anti-PEG-IgM in human serum was detected by sandwich ELISA, as shown in Figure 13.

Solid lipid nanoparticles encapsulating the tumor suppressor gene PTEN DNA were prepared, and their binding activity with anti-PEG-IgM was investigated. As shown in Figure 13, HO-LNP/DNA showed a lower binding to PEG-IgM. That is, after encapsulating DNA, HO-LNP still retains a lower binding activity with PEG-IgM.

### Example 14

This example is used to illustrate that hydroxyl PEG can reduce complement activation by circumventing the binding to natural anti-PEG-IgM in human serum.

The complement activation capability of methoxy or hydroxyl PEG nanoformulations was detected by sheep erythrocyte hemolysis assay as follows: sheep erythrocytes were washed twice with PBS and adjusted to a concentration of 4%. Hemolysin was diluted 1:1000, mixed with 4% sheep erythrocytes, and incubated at 37°C for 30 min to sensitize erythrocytes. Human serum was incubated with PEG nanoformulations at 37°C for 30 min to deplete the complement. Then, the sensitized sheep erythrocytes were added, incubated at 37°C for 30 min, and finally centrifuged to remove unlysed sheep erythrocytes. The OD542nm absorbance in the supernatant was detected, as shown in Figure 14.

When the antigen binds to the antibody, the complement proteins in the blood are activated and depleted through the classical pathway, resulting in a hypersensitivity and accelerating the phagocytosis of the mononuclear macrophage system, thereby shortening the blood circulation time. The clinical infusion reaction of liposomal doxorubicin and the allergic reaction of COVID-19 mRNA vaccine (LNP encapsulating SARS-CoV-2 mRNA) are caused by complement activation, so the evaluation of the complement activation capability of hydroxyl-modified PEGylated nanoformulations is an important indicator for predicting clinical allergic reactions. The sheep erythrocyte hemolysis assay was used to test the complement activation capability of the formulation. Sheep erythrocytes were incubated with hemolysin to obtain sensitized sheep erythrocytes, which caused complement activation when encountering complement proteins, and produced a membrane attack complex (C5b-9), which led to the rupture of red blood cells and the release of hemoglobin. Differences in complement content between samples can be compared by measuring the absorbance values in the supernatant after centrifuging the remaining red blood cells. The serum of healthy people was first incubated with different PEGylated formulations at 37°C for 30 min to activate complement, and then the remaining complement content in the serum was detected by sensitized sheep erythrocytes. As shown in Figure 14, the complement activation capability of hydroxyl PEG-modified nanoformulation was lower than that of methoxy PEG-modified nanoformulation, resulting in stronger hemolysis of sheep erythrocytes caused by the remaining complement.

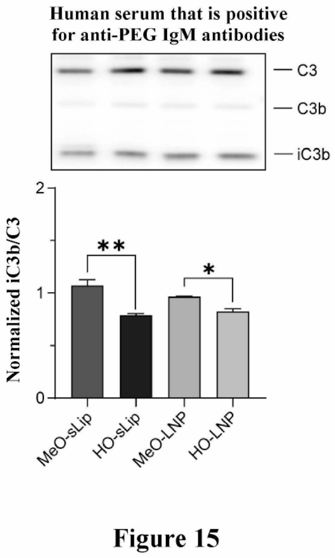

Furthermore, the method of example 3 was used to detect the complement activation of hydroxyl PEG-modified nanoformulations in human serum in the presence of pre-existing anti-PEG antibodies. As shown in Figure 15, the degree of complement activation of hydroxy PEG-modified liposomes or LNPs in anti-PEG antibody-positive human serum is lower than that of methoxy PEG-modified liposomes or LNPs. Combined with the results of sheep erythrocyte hemolysis assay, it can be concluded that hydroxyl PEG-modified nanoformulations can reduce complement activation by circumventing the binding to natural anti-PEG-IgM in human blood, thereby alleviating clinical allergic reactions.

The protection content of the present invention is not limited to the above examples. Changes and substitutions that occur to a person skilled in the art are included in the present invention without departing from the spirit and scope of the invention, and the scope of protection is defined by the following claims.

## Claims

1. A use of a terminal hydroxyl-modified PEGylated nanocarrier in the preparation of a drug that circumvents pre-existing anti-PEG antibodies in the human body, wherein the PEGylated nanocarrier is selected from one or more of liposomes, polymer nanoparticles, micelles and lipid nanoparticles.

2. The use of claim 1, wherein the drug includes one or more of small molecule drugs, protein drugs and nucleic acid drugs.

3. The use of claim 2, wherein the nucleic acid drugs are selected from one or more of small interfering ribonucleic acids (siRNA), deoxyribonucleic acids (DNA) and messenger ribonucleic acids (mRNA).

4. The use of any one of claims 1-3, wherein the molecular weight of the terminal hydroxyl-modified PEG is 500-10000 Da; and/or wherein the ratio of PEG modification in the liposomes and lipid nanoparticles is 0.5mol%-10mol%, and the ratio of PEG modification in the micelles and polymer nanoparticles is 0.5mol%-100mol%.

5. The use of any one of claims 1-3, wherein the terminal hydroxyl-modified PEG has a hydroxyl group at one end, and a polymer or lipid at the other end.

6. The use of any one of claims 1-3, wherein the PEGylated nanocarrier includes one or more of hydroxyl polyethylene glycol polylactic acid-glycolic acid copolymer (HO-PEG-PLGA), hydroxyl polyethylene glycol polylactic acid copolymer (HO-PEG-PLA), hydroxyl polyethylene glycol-distearoylphosphatidylethanolamine (HO-PEG-DSPE) and hydroxyl polyethylene glycol-dimyristoylglycerol (HO-PEG-DMG).

7. A nanoformulation for human use, wherein the nanoformulation comprises an active ingredient and a terminal hydroxyl-modified PEGylated nanocarrier, the nanocarrier is selected from one or more of liposomes, polymer nanoparticles, lipid nanoparticles and micelles, and the active ingredient is selected from one or more of small molecule drugs, protein drugs and nucleic acid drugs.

8. The nanoformulation for human use of claim 7, wherein the molecular weight of the terminal hydroxyl-modified PEG is 500-10000 Da; and/or wherein the ratio of PEG modification in the liposomes and lipid nanoparticles is 0.5mol%-10mol%, and the ratio of PEG modification in the micelles and polymer nanoparticles is 0.5mol%-100mol%.

9. The nanoformulation for human use of claim 7 or 8, wherein the nucleic acid drugs are selected from one or more of siRNA, DNA and mRNA.

10. The nanoformulation for human use of claim 7 or 8, wherein the terminal hydroxyl-modified PEG has a hydroxyl group at one end, and a polymer or lipid at the other end.

11. The nanoformulation for human use of claim 7 or 8, wherein the PEGylated nanocarrier includes one or more of hydroxyl polyethylene glycol polylactic acid-glycolic acid copolymer (HO-PEG-PLGA), hydroxyl polyethylene glycol polylactic acid copolymer (HO-PEG-PLA), hydroxyl polyethylene glycol-distearoylphosphatidylethanolamine (HO-PEG-DSPE) and hydroxyl polyethylene glycol-dimyristoylglycerol (HO-PEG-DMG).

12. A method for treating a disease by circumventing pre-existing anti-PEG antibodies in the human body, wherein the method includes administering a nanoformulation to a human subject in need thereof, wherein the nanoformulation comprises an active ingredient and a terminal hydroxyl-modified PEGylated nanocarrier, the nanocarrier is selected from one or more of liposomes, polymer nanoparticles, lipid nanoparticles and micelles, and the active ingredient is selected from one or more of small molecule drugs, protein drugs and nucleic acid drugs.

13. The method of claim 12, wherein the molecular weight of the terminal hydroxyl-modified PEG is 500-10000 Da; and/or wherein the ratio of PEG modification in the liposomes and lipid nanoparticles is 0.5mol%-10mol%, and the ratio of PEG modification in the micelles and polymer nanoparticles is 0.5mol%-100mol%.

14. The method of claim 12 or 13, wherein the nucleic acid drugs are selected from one or more of siRNA, DNA and mRNA.

15. The method of claim 12 or 13, wherein the terminal hydroxyl-modified PEG has a hydroxyl group at one end, and a polymer or lipid at the other end.

16. The method of claim 12 or 13, wherein the PEGylated nanocarrier includes one or more of hydroxyl polyethylene glycol polylactic acid-glycolic acid copolymer (HO-PEG-PLGA), hydroxyl polyethylene glycol polylactic acid copolymer (HO-PEG-PLA), hydroxyl polyethylene glycol-distearoylphosphatidylethanolamine (HO-PEG-DSPE) and hydroxyl polyethylene glycol-dimyristoylglycerol (HO-PEG-DMG).
